# EUROPEAN PATENT APPLICATION

(11) **EP 2 116 529 A1**
(43) Date of publication of application: **11.11.2009**
(21) Application number: 07859896.8
(22) Date of filing: 20.12.2007
(51) Int. Cl.: C07D 201/04, C07C 231/10, C07D 211/76, C07D 223/10, C07D 225/02, C07D 227/087

(54) **PROCESS FOR PRODUCTION OF AMIDE OR LACTAM**

(30) Priority: 28.12.2006 JP 2006353956
(71) Applicant: Daicel Chemical Industries, Ltd., Kita-ku Osaka-shi Osaka 530-0001 (JP)
(72) Inventor: ISHII, Yasutaka, Takatsuki-shi Osaka 569-1112 (JP); IWAHAMA, Takahiro, Himeji-shi Hyogo 671-1283 (JP); NAKANO, Tatsuya, Himeji-shi Hyogo 671-1283 (JP)
(74) Representative: Grünecker, Kinkeldey, Stockmair & Schwanhäusser Anwaltssozietät
(86) International application number: PCT/JP2007/074499
(87) International publication number: WO 2008/081726

(57) **Abstract**

Disclosed is a process for producing an amide or lactam in a high yield in a simple manner by allowing a rearrangement reaction of an oxime compound to proceed without causing large amounts of by-products such as ammonium sulfate.

Specifically, disclosed is a process for producing an amide or lactam in which a corresponding amide or lactam is produced by rearranging an oxime compound in the presence of a compound containing a structure represented by following Formula (1):

-Z-X (1)

wherein Z represents P, N, S, B, or Si atom; and X represents a leaving group, where Z is bonded to one or more atoms or groups besides X. In the process, X may be a halogen atom.

## Description

### Technical Field

The present invention relates to processes for producing lactams or amides which are useful typically as raw materials for pharmaceutical drugs (medicines), agricultural chemicals, dyestuffs, and polyamides and as solvents. More specifically, it relates to processes for producing the amides or lactams through rearrangement reactions of oxime compounds.

### Background Art

Techniques for producing corresponding lactams from raw material cyclic oxime compounds through "Beckmann rearrangement" are industrially very important. The production of these compounds has been conducted by a process of acting fuming sulfuric acid in a stoichiometric amount or more, but this process raises an issue of by-production of large amounts of ammonium sulfate to be treated.

In view of the problem, there has been proposed the use of an aromatic compound having two or more electro-withdrawing groups and a halo group acting as a leaving group, such as 2,4,6-trichloro-1,3,5-triazine, as a Beckmann rearrangement catalyst that places less load on the environment (see Non-patent Document 1 and Patent Document 1). However, little has yet been known about catalysts that induce Beckmann rearrangement, and demands have been made to develop novel compounds acting as such catalysts.

Non-patent Document 1: J. AM. CHEM. SOC. 2005, 127, 11240-11241
Patent Document 1: Japanese Unexamined Patent Application Publication (JP-A) No. 2006-219470

### Disclosure of Invention

### Problems to be Solved by the Invention

Accordingly, an object of the present invention is to provide a process for producing an amide or lactam in a simple manner and in a high yield by allowing a rearrangement reaction of an oxime compound to proceed without causing large amounts of by-products such as ammonium sulfate.

### Means for Solving the Problems

After intensive investigations to achieve the object, the present inventors have found that the object can be achieved by using a compound containing a specific structure as a constituent. The present invention has been made based on these findings.

Specifically, the present invention provides a process for producing an amide or lactam, the process comprising the step of carrying out rearrangement of an oxime compound in the presence of a compound containing a structure represented by following Formula (1):

-Z-X (1)

wherein Z represents phosphorus (P), nitrogen (N), sulfur (S), boron (B), or silicon (Si) atom; and X represents a leaving group, where Z is bonded to one or more atoms or groups besides X,
to give a corresponding amide or lactam.

In the process, X is preferably a halogen atom. Advantages

According to the present invention, the problems of removal and disposal of by-products occurring in known processes for producing amides or lactams can be avoided, and amides or lactams can be produced in a simple manner and in a high yield, because rearrangement reactions of oximes can be carried out without causing large amounts of by-products such as ammonium sulfate.

### Best Modes for Carrying Out the Invention

According to present invention, the compound containing a structure represented by Formula (1) as a molecular constituent acts as a catalyst to allow a rearrangement reaction of an oxime compound to proceed rapidly and efficiently to thereby give a corresponding amide or lactam in a high yield.

In Formula (1), the leaving group as X can be any common leaving functional group (of which a group that is capable of leaving as X-H is preferred), and examples thereof include halogen atoms (fluorine atom, chlorine atom, bromine atom, and iodine atom), -OR groups (wherein R represents an organic group), carboxyl group, amino groups, and sulfonyloxy groups. Among them, halogen atoms are preferably used. Though not especially limited, preferred examples of the organic group as R include alkyl groups, haloalkyl groups, and groups represented by following Formula (2):

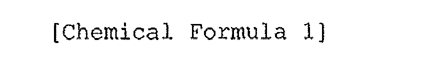

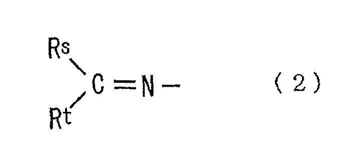

wherein R^{s} and R^{t} are the same as or different from each other and each represent a hydrocarbon group, where R^{s} and R^{t} may be combined to form a nonaromatic ring with the carbon atom to which R^{s} and R^{t} are bonded.

Though not especially limited, exemplary hydrocarbon groups as R^{s} and R^{t} include aliphatic chain groups including alkyl groups, alkenyl groups, and alkynyl groups, each having about 1 to about 10 carbon atoms; as well as cycloalkyl groups, aryl groups, and aralkyl groups. Exemplary nonaromatic rings formed by R^{s} and R^{t} with the carbon atom to which R^{t} and R^{s} are bonded include cycloalkyl groups. In this case, the group represented by Formula (2) is a cycloalkylideneamino group. The organic group R, if being a group represented by Formula (2), can be a group corresponding to the oxime compound used as a raw material (a group corresponding to the oxime compound, except for removing -OH group therefrom).

Exemplary alkyl groups as R include linear or branched-chain alkyl groups having 1 to 10 carbon atoms, such as methyl, ethyl, propyl, isopropyl, butyl, isobutyl, s-butyl, t-butyl, pentyl, and hexyl. Exemplary haloalkyl groups as R include groups corresponding to the alkyl groups, except with one or more halogen atoms, such as fluorine, chlorine, bromine, and iodine, being substituted. The haloalkyl groups may each have a halogenated aryl group as a substituent. Of haloalkyl groups, preferred are fluorinated alkyl groups substituted with fluorine atom, of which more preferred are fluorine-containing branched-chain aliphatic groups represented by Formula (3a); fluorine-containing linear aliphatic groups represented by Formula (3b); and fluorine-containing aliphatic chain groups bonded with a fluorophenyl group, such as a group represented by Formula (3c). When the group represented by R is a fluorinated alkyl group, the fluorinated alkyl group is often a group corresponding to a fluorine-containing alcohol mentioned later:

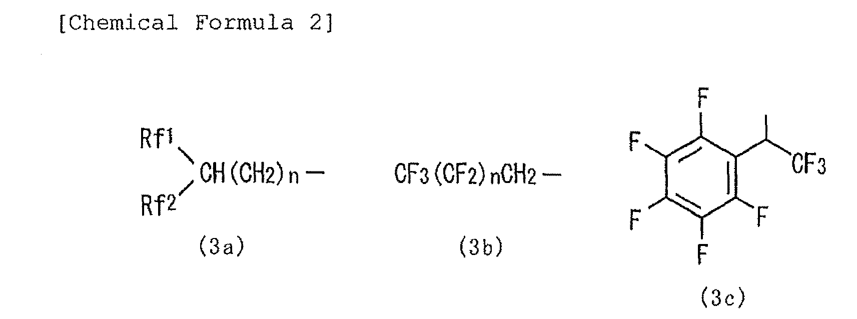

wherein Rf¹ and Rf² may be the same as or different from each other and each represent a perfluoroalkyl group having 1 to 8 carbon atoms; and "n" denotes an integer of from 0 to 10.

The compound containing a structure represented by Formula (1) is not especially limited, as long as a compound containing one or more of the structure per molecule, and may be a cyclic compound or an acyclic compound.

Exemplary compounds containing a structure represented by Formula (1) for use herein include phosphazene compounds (phosphazene derivatives) represented by following Formula (1a), phosphoric ester compounds (phosphoric ester derivatives) represented by Formula (1b), phosphine compounds (phosphine derivatives) represented by Formula (1c), imide compounds (imide derivatives) represented by Formula (1d), sulfonyl or sulfinyl compounds (sulfonyl or sulfinyl derivatives) represented by Formula (1e), silane compounds (silane derivatives) represented by Formula (1f), and cyclic compounds represented by Formula (1g) and containing silicon atom as a ring constituent:

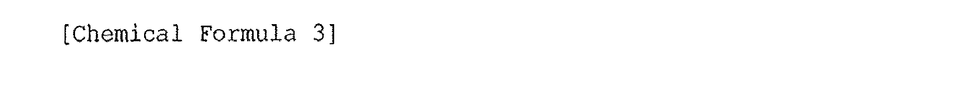

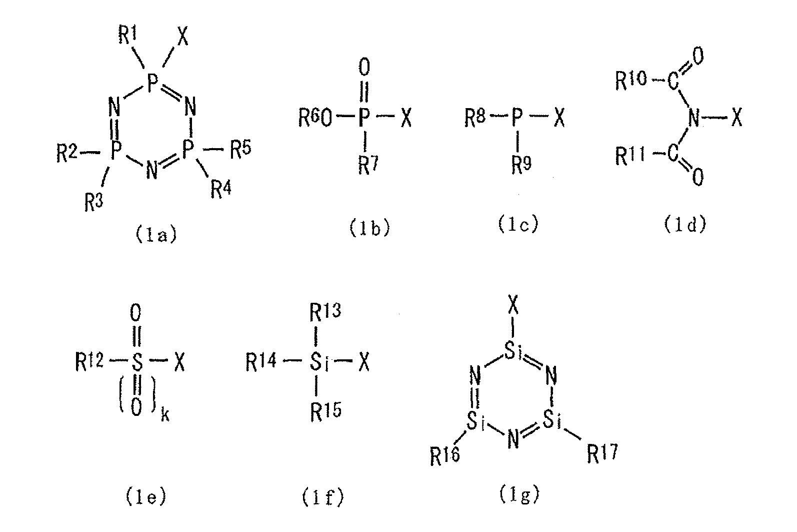

wherein X is as defined above; "k" denotes 0 or 1; R¹, R², R³, R⁴, R⁵, R⁶, R⁷, R⁸, R⁹, R¹⁰, R¹¹, R¹², R¹³, R¹⁴, R¹⁵, R¹⁶, and R¹⁷ are the same as or different from one another and are each a hydrogen atom, halogen atom, alkyl group, haloalkyl group, aryl group, aralkyl group, cycloalkyl group, hydroxyl group, alkoxy group, aryloxy group, haloalkoxy group, mercapto group, carboxyl group, substituted oxycarbonyl group, acyl group, acyloxy group, nitro group, sulfo group, cyano group, amino group, oxyamino group, or another organic group. In Formula (1a), a pair of R² and R³ and/or a pair of R⁴ and R⁵ may be combined to form a ring with the adjacent phosphorus atom, respectively. In Formula (1b), R⁶ and R⁷ may be combined to form a ring with the adjacent oxygen atom and phosphorus atom. In Formula (1c), R⁸ and R⁹ may be combined to form a ring with the adjacent phosphorus atom. In Formula (1d), R¹⁰ and R¹¹ may be combined to form a ring with the adjacent two carbon atoms and nitrogen atom. In Formula (1f), at least two of R¹³, R¹⁴, and R¹⁵ may be combined to form a ring with the adjacent silicon atom.

As R¹ to R¹⁷, exemplary halogen atoms include iodine, bromine, chlorine, and fluorine atoms. Exemplary alkyl groups include linear or branched-chain alkyl groups having about 1 to about 30 carbon atoms, such as methyl, ethyl, propyl, isopropyl, butyl, isobutyl, s-butyl, t-butyl, hexyl, decyl, dodecyl, tetradecyl, and hexadecyl groups, of which those having 1 to 20 carbon atoms are preferred, and those having 1 to 6 carbon atoms are more preferred. Exemplary haloalkyl groups include groups corresponding to these alkyl groups, except with one or more halogen atoms, such as fluorine, chlorine, bromine, and iodine atoms, being substituted.

Exemplary aryl groups include phenyl, tolyl, xylyl, and naphthyl groups; exemplary aralkyl groups include benzyl, 2-phenylethyl, 1-phenylethyl, and trityl groups; and exemplary cycloalkyl groups include cyclopentyl and cyclohexyl groups. Exemplary alkoxy groups include alkoxy groups having about 1 to about 30 carbon atoms, such as methoxy, ethoxy, isopropoxy, butoxy, t-butoxy, hexyloxy, octyloxy, decyloxy, dodecyloxy, tetradecyloxy, and octadecyloxy groups, of which those having 1 to 20 carbon atoms are preferred, and those having 1 to 6 carbon atoms are more preferred. Exemplary aryloxy groups include phenyloxy group.

Exemplary haloalkoxy groups include groups corresponding to fluorine-containing branched-chain aliphatic alcohols, except for removing hydrogen atom therefrom [groups corresponding to the fluorine-containing branched-chain aliphatic groups represented by Formula (3a), except with oxygen atom bonded thereto], such as hexafluoroisopropyloxy group (2,2,2-trifluoro-1-trifluoromethylethoxy group); groups corresponding to fluorine-containing linear aliphatic alcohols (fluorine-containing primary alcohols), except for removing hydrogen atom therefrom [groups corresponding to the fluorine-containing linear aliphatic groups represented by Formula (3b), except with oxygen atom bonded thereto]; and groups corresponding to fluorine-containing aliphatic chain groups bonded with a fluorophenyl group, except with oxygen atom bonded thereto, such as a group corresponding to the group represented by Formula (3c), except with oxygen atom bonded thereto.

Exemplary substituted oxycarbonyl groups include alkoxy-carbonyl groups whose alkoxy moiety having 1 to 30 carbon atoms, such as methoxycarbonyl, ethoxycarbonyl, isopropoxycarbonyl, butoxycarbonyl, t-butoxycarbonyl, hexyloxycarbonyl, decyloxycarbonyl, and hexadecyloxycarbonyl groups, of which alkoxy-carbonyl groups whose alkoxy moiety having 1 to 20 carbon atoms are preferred, and alkoxy-carbonyl groups whose alkoxy moiety having 1 to 6 carbon atoms are more preferred; cycloalkyloxycarbonyl groups such as cyclopentyloxycarbonyl and cyclohexyloxycarbonyl groups, of which cycloalkyloxycarbonyl groups having 3 to 20 members are preferred, and cycloalkyloxycarbonyl groups having 3 to 15 members are more preferred; aryloxycarbonyl groups such as phenyloxycarbonyl and naphthyloxycarbonyl groups, of which aryloxy-carbonyl groups whose aryloxy moiety having 6 to 20 carbon atoms are preferred; aralkyloxycarbonyl groups such as benzyloxycarbonyl group, of which aralkyloxycarbonyl groups whose aralkyloxy moiety having 7 to 21 carbon atoms are preferred.

Exemplary acyl groups include aliphatic saturated or unsaturated acyl groups including aliphatic acyl groups having 1 to 30 carbon atoms, such as formyl, acetyl, propionyl, butyryl, isobutyryl, valeryl, pivaloyl, hexanoyl, octanoyl, decanoyl, lauroyl, myristoyl, palmitoyl, and stearoyl groups, of which aliphatic acyl groups having 1 to 20 carbon atoms are preferred, and aliphatic acyl groups having 1 to 6 carbon atoms are more preferred; acetoacetyl group; alicyclic acyl groups including cycloalkanecarbonyl groups such as cyclopentanecarbonyl and cyclohexanecarbonyl groups; and aromatic acyl groups such as benzoyl and naphthoyl groups.

Exemplary acyloxy groups include aliphatic saturated or unsaturated acyloxy groups including aliphatic acyloxy groups having 1 to 30 carbon atoms, such as formyloxy, acetyloxy, propionyloxy, butyryloxy, isobutyryloxy, valeryloxy, pivaloyloxy, hexanoyloxy, octanoyloxy, decanoyloxy, lauroyloxy, myristoyloxy, palmitoyloxy, and stearoyloxy groups, of which aliphatic acyloxy groups having 1 to 20 carbon atoms are preferred; acetoacetyloxy group; alicyclic acyloxy groups including cycloalkanecarbonyloxy groups such as cyclopentanecarbonyloxy and cyclohexanecarbonyloxy groups; and aromatic acyloxy groups such as benzoyloxy and naphthoyloxy groups. Examples of the other organic group include the groups represented by Formula (2).

Though not especially limited, the rings formed by the pair or R² and R³ and the pair of R⁴ and R⁵, respectively, with the adjacent phosphorus atom, the ring formed by R⁶ and R⁷ with the adjacent oxygen atom and phosphorus atom, the ring formed by R⁸ and R⁹ with the adjacent phosphorus atom, the ring formed by R¹⁰ and R¹¹ with the adjacent two carbon atoms and nitrogen atom, and the ring formed by at least two of R¹³, R¹⁴, and R¹⁵ with the adjacent silicon atom are generally heterocyclic rings each having about 3 to 12 members. These rings may each have one or more substituents bonded thereto. These rings may have one or more other rings fused thereto. Exemplary substituents herein include alkyl groups, haloalkyl groups, hydroxyl group, alkoxy groups, carboxyl group, substituted oxycarbonyl groups, acyl groups, acyloxy groups, nitro group, cyano group, amino groups, and halogen atoms. Exemplary rings to be fused include aromatic hydrocarbon rings such as benzene ring; aromatic heterocyclic rings such pyridine ring; nonaromatic hydrocarbon rings (aliphatic rings) such as cyclohexane ring; and nonaromatic heterocyclic rings such as tetrahydrofuran ring.

In the compounds represented by Formulae (1a) to (1g), the groups R¹ to R¹⁷ can be leaving groups as with X, of which halogen atoms and -OR groups (where R represents an organic group) are preferred. A phosphazene compound represented by Formula (1a), if at least R² and R⁴ are leaving groups as with X, be a cyclic compound containing three structures represented by Formula (1) per one molecule. Likewise, a cyclic compound represented by Formula (1g) and containing silicon atom as a ring constituent, if R¹⁶ and R¹⁷ are leaving groups as with X, be a cyclic compound containing three structures represented by Formula (1) per one molecule.

In Formula (1b), R⁶ is especially preferably an alkyl group, haloalkyl group, aryl group, aralkyl group, or cycloalkyl group. R⁷ is preferably a similar leaving group to X [of which a halogen atom or -OR group, wherein R represents an organic group, is preferred], or an -OR⁶ group. In Formula (1c), R⁸ and R⁹ are each especially preferably an alkyl group, haloalkyl group, aryl group, aralkyl group, cycloalkyl group, or similar leaving group to X [of which a halogen atom or -OR group, wherein R represents an organic group, is preferred].

In Formula (1d), it is especially preferred that R¹⁰ and R¹¹ are combined to form a ring with the adjacent two carbon atoms and nitrogen atom. The ring may have one or more substituents bonded thereto and may have one or more other rings fused therewith. In Formula (1e), R¹² is especially preferably an alkyl group, haloalkyl group, aryl group, aralkyl group, cycloalkyl group, or similar leaving group to X [of which a halogen atom or -OR group, wherein R represents an organic group, is preferred]. In Formula (1f), R¹³, R¹⁴, and R¹⁵ are each especially preferably an alkyl group, haloalkyl group, aryl group, aralkyl group, cycloalkyl group, or similar leaving group to X [of which a halogen atom or -OR group, wherein R represents an organic group, is preferred].

Specific examples of phosphazene compounds represented by Formula (1a) include halophosphazene derivatives such as hexachlorophosphazene (a compound wherein X, R¹, R², R³, R⁴, and R⁵ are each chlorine (Cl)), hexafluorophosphazene (a compound wherein X, R¹, R², R³, R⁴, and R⁵ are each fluoride (F)), and hexabromophosphazene (a compound wherein X, R¹, R², R³, R⁴, and R⁵ are each bromine (Br)); and compounds represented by following Formula (1a-1):

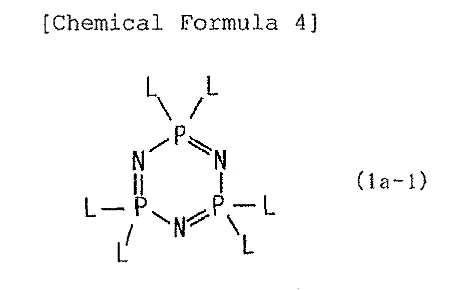

wherein Ls are each a group represented by following Formula (a), (b) or (c): 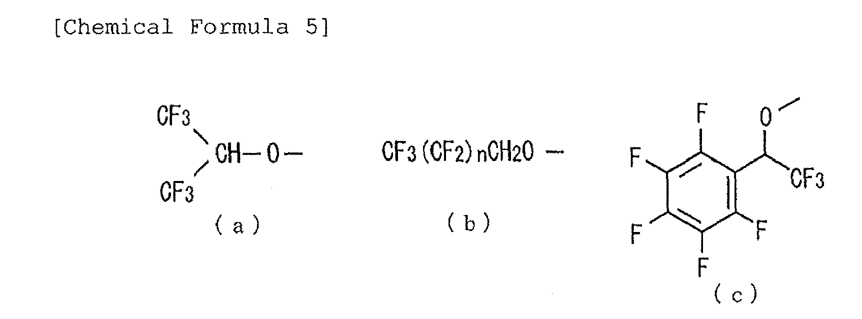 In Formula (b), "n" denotes an integer of from 0 to 10.

Exemplary phosphoric ester compounds represented by Formula (1b) include dimethyl chlorophosphate, diethyl chlorophosphate, 2-chloro-1,3,2-dioxaphospholane-2-oxide, methyl dichlorophosphate, ethyl dichlorophosphate, diphenyl chlorophosphate, 1,2-phenylene phosphorochloridate, phenyl dichlorophosphate, and compounds represented by following Formula (1b-1):

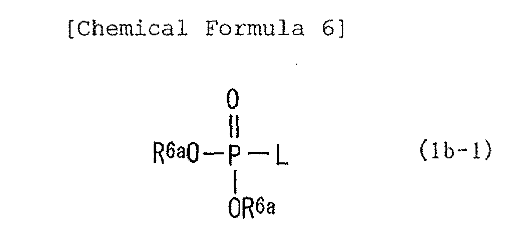

wherein R^{6a} represents methyl group, ethyl group, or phenyl group; and L is as defined above.

Specific examples of phosphine compounds represented by Formula (1c) include halophosphine derivatives such as chlorodimethylphosphine, chlorodiethylphosphine, chlorodipropylphosphine, chlorodiphenylphosphine, dichloroethylphosphine, dichlorobutylphosphine, and dichlorohexylphosphine; and compounds represented by following Formula (1c-1):

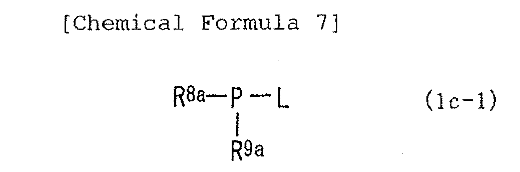

wherein R^{8a} and R^{9a} are each methyl group, ethyl group, or phenyl group; and L is as defined above.

Specific examples of imide compounds represented by Formula (1d) include succinimide derivatives including N-halosuccinimide derivatives such as N-chlorosuccinimide, N-bromosuccinimide, N-iodosuccinimide, and N-fluorosuccinimide; phthalimide derivatives including N-halophthalimide derivatives such as N-chlorophthalimide, N-bromophthalimide, N-iodophthalimide, and N-fluorophthalimide; maleimide derivatives including N-halomaleimide derivatives such as N-chloromaleimide, N-bromomaleimide, N-iodomaleimide, and N-fluoromaleimide; isocyanuric acid derivatives including isocyanuric halide derivatives such as trichloroisocyanuric acid (isocyanuric chloride) and dichloroisocyanuric acid sodium salt; and hydantoin derivatives including halohydantoin derivatives such as 1,3-dichloro-5,5-dimethylhydantoin and 1,3-dibromo-5,5-dimethylhydantoin.

Specific examples of sulfonyl or sulfinyl compounds represented by Formula (1e) include sulfonyl halide derivatives such as methanesulfonyl chloride, ethanesulfonyl chloride, propanesulfonyl chloride, trichloromethanesulfonyl chloride, trifluoromethanesulfonyl chloride, benzenesulfonyl chloride, toluenesulfonyl chloride, nitrobenzenesulfonyl chloride, chlorobenzenesulfonyl chloride, fluorobenzenesulfonyl chloride, and naphthalenesulfonyl chloride; sulfanyl chloride; and thionyl chloride.

Specific examples of silane compounds represented by Formula (1f) include halosilane derivatives such as chlorotriphenylsilane, dichlorodiphenylsilane, and phenyltrichlorosilane.

Specific examples of cyclic compounds represented by Formula (1g) and containing silicon atom as a ring constituent include a compound represented by following Formula (1g-1):

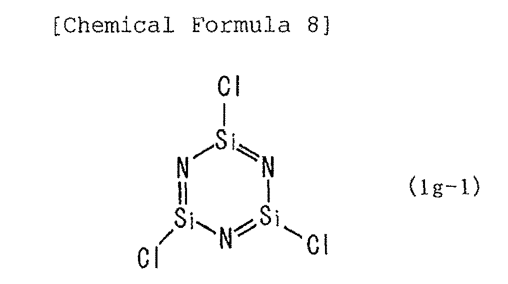

Among them, phosphazene compounds represented by Formula (1a), phosphoric ester compounds represented by Formula (1b), and imide compounds represented by Formula (1d) are preferably used.

When the compound containing a structure represented by Formula (1) is a compound having an -OR group as X, the compound may be previously prepared before subjected to the reaction, but such compound having an -OR group as X can also be formed by incorporating a corresponding compound having a halogen atom as X and a compound that generates an RO⁻ ion into the reaction system for producing an amide or lactam, to allow a substitution reaction between the halogen atom and the -OR group within the reaction system. Though not especially limited, the compound that generates an RO⁻ ion is often an after-mentioned fluorine-containing alcohol used as a promoter, or an oxime compound used as a raw material. Specifically, embodiments of the present invention in which X is an -OR group include embodiments in which a compound containing a structure represented by Formula (1) wherein X is a halogen atom is used, and this compound is allowed to react with a fluorine-containing alcohol in the reaction system to give a compound having a haloalkoxy group as a substituent or the compound is allowed to react with an oxime compound in the reaction system to give a compound having, as a substituent, a group which corresponds to the oxime compound, except for removing hydrogen atom therefrom (for example, a cycloalkylideneaminooxy group).

The amount of such compounds containing a structure represented by Formula (1) is, for example, from about 0.0001 to about 1 mole, preferably from about 0.0005 to about 0.5 mole, and more preferably from about 0.001 to about 0.2 mole, per 1 mole of the oxime compound. Each of different compounds containing a structure represented by Formula (1) may be used alone or in combination.

The compound containing a structure represented by Formula (1) for use in the present invention shows a high catalytic activity in a Beckmann rearrangement reaction. Though remaining unknown, this is probably because the rearrangement reaction proceeds through an intermediate in which oxygen atom in the oxime moiety of the substrate oxime compound is combined with the heteroatom Z (P, N, S, B, or Si atom) in the compound containing a structure represented by Formula (1), and in this stage, the leaving group X in Formula (1) is combined with proton in the oxime moiety of the oxime compound and leaves as X-H.

The oxime compound for use as a raw material in the present invention is not especially limited and can be appropriately selected according to an amide or lactam to be produced. Exemplary oxime compounds include compounds represented by following Formula (4) or Formula (5):

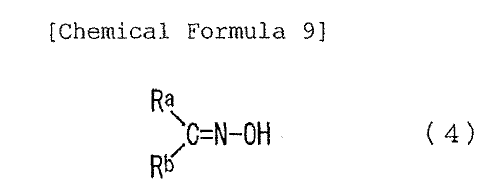

wherein R^{a} and R^{b} each represent an organic group, wherein at least one of R^{a} and R^{b} may be a hydrogen atom,

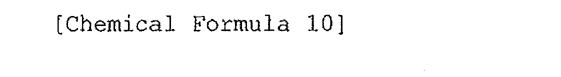

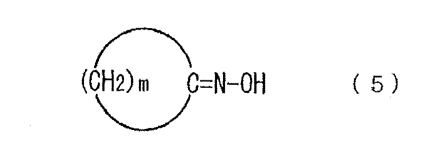

wherein "m" denotes an integer of 2 or more.

Exemplary organic groups as R^{a} and R^{b} include methyl, ethyl, propyl, isopropyl, butyl, isobutyl, s-butyl, t-butyl, pentyl, isopentyl, hexyl, isohexyl, heptyl, octyl, nonyl, decyl, dodecyl, pentadecyl, and other alkyl groups (e.g., alkyl groups having 1 to 20 carbon atoms, of which alkyl groups having 1 to 12 carbon atoms are preferred, and alkyl groups having 2 to 8 carbon atoms are more preferred); vinyl, allyl, 1-propenyl, 1-butenyl, 1-pentenyl, 1-octenyl, and other alkenyl groups (e.g., alkenyl groups having 2 to 20 carbon atoms, of which alkenyl groups having 2 to 12 carbon atoms are preferred, and alkenyl groups having 2 to 8 carbon atoms are more preferred); ethynyl, 1-propynyl, and other alkynyl groups (e.g., alkynyl groups having 2 to 20 carbon atoms, of which alkynyl groups having 2 to 12 carbon atoms are preferred, and alkynyl groups having 2 to 8 carbon atoms are more preferred); cyclopropyl, cyclobutyl, cyclopentyl, cyclohexyl, cycloheptyl, cyclooctyl, cyclododecyl, and other cycloalkyl groups (e.g., cycloalkyl groups having 3 to 20 carbon atoms, of which cycloalkyl groups having 3 to 15 carbon atoms are preferred); cyclopentenyl, cyclohexenyl, cyclooctenyl, and other cycloalkenyl groups (e.g., cycloalkenyl groups having 3 to 20 carbon atoms, of which cycloalkenyl groups having 3 to 15 carbon atoms are preferred); phenyl, naphthyl, and other aryl groups; benzyl, 2-phenylethyl, 3-phenylpropyl, and other aralkyl groups; and 2-pyridyl, 2-quinolyl, 2-furyl, 2-thienyl, 4-piperidinyl, and other aromatic or nonaromatic heterocyclic groups. These organic groups may each have one or more substituents within ranges not adversely affecting the reaction. Exemplary substituents include halogen atoms, oxo group, hydroxyl group, mercapto group, substituted oxy groups (e.g., alkoxy groups, aryloxy groups, and acyloxy groups), substituted thio groups, carboxyl group, substituted oxycarbonyl groups, substituted or unsubstituted carbamoyl groups, cyano group, nitro group, substituted or unsubstituted amino groups, alkyl groups, alkenyl groups, alkynyl groups, cycloalkyl groups, cycloalkenyl groups, aryl groups (e.g., phenyl and naphthyl groups), aralkyl groups, and heterocyclic groups.

Specific examples of oxime compounds represented by Formula (4) include acetaldehyde oxime, acetone oxime, 2-butanone oxime, 2-pentanone oxime, 3-pentanone oxime, 1-cyclohexyl-1-propanone oxime, benzaldehyde oxime, acetophenone oxime, benzophenone oxime, and 4'-hydroxyacetophenone oxime.

The ring in Formula (5) may have one or more substituents bonded thereto and may have one or more rings fused herewith. The repetition number "m" is, for example, from about 2 to about 30, preferably from about 4 to about 20, and more preferably from about 5 to about 14. Exemplary cyclic oxime compounds represented by Formula (5) include cyclopropanone oxime, cyclobutanone oxime, cyclohexanone oxime, cycloheptanone oxime, cyclooctanone oxime, cyclononanone oxime, cyclodecanone oxime, cyclododecanone oxime, cyclotridecanone oxime, cyclotetradecanone oxime, cyclopentadecanone oxime, cyclohexadecanone oxime, cyclooctadecanone oxime, and cyclononadecanone oxime. Exemplary substituents which may be bonded to the ring are as with the exemplified substituents which the organic groups may have.

Preferred embodiments of the present invention include an embodiment in which the oxime compound is an oxime compound represented by Formula (4), X in Formula (1) is an -OR group, and R is a group represented by following Formula (4a); and an embodiment in which the oxime compound is an oxime compound represented by Formula (5), X in Formula (1) is an -OR group, and R is a group represented by following Formula (5a):

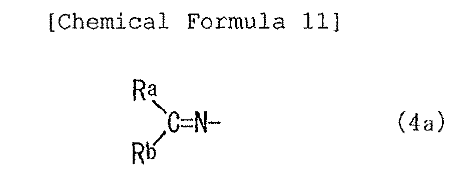

wherein R^{a} and R^{b} each represent an organic group, wherein at least one of R^{a} and R^{b} may be a hydrogen atom,

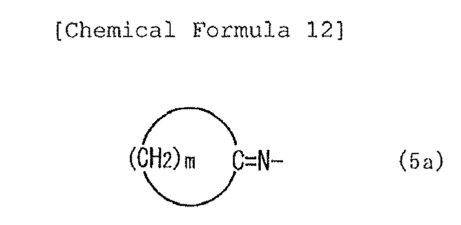

wherein "m" denotes an integer of 2 or more.

Each of different oxime compounds may be used alone or in combination.

The rearrangement reaction of the oxime compound is carried out in the presence of, or in the absence of, a solvent. The solvent is not specifically limited, as long as being inert (inactive) under reaction conditions, and examples thereof include organic acids such as acetic acid, propionic acid, and trifluoroacetic acid; nitriles such as acetonitrile, propionitrile, and benzonitrile; amides such as formamide, acetamide, dimethylformamide (DMF), and dimethylacetamide; aliphatic hydrocarbons such as hexane, heptane, octane, and cyclododecane; aromatic hydrocarbons such as benzene, toluene, and xylenes; halogenated hydrocarbons such as chloroform, dichloromethane, dichloroethane, carbon tetrachloride, chlorobenzene, and trifluoromethylbenzene; nitro compounds such as nitrobenzene, nitromethane, and nitroethane; esters such as ethyl acetate and butyl acetate; fluorine-containing alcohols such as hexafluoroisopropyl alcohol and trifluoroethanol; and mixtures of these solvents.

The reaction temperature can be appropriately set according to the types typically of the oxime compound, catalyst, and solvent to be used, and is not especially limited. It is for example, from about 0°C to about 250°C, preferably from about 25°C to about 150°C, and more preferably from about 40°C to about 120°C. The reaction may be carried out in an atmosphere of an inert gas such as nitrogen or argon gas or can be carried out in an air atmosphere or in an oxygen atmosphere. The reaction in the present invention is especially preferably carried out in an air atmosphere under reflux conditions.

The use of a fluorine-containing alcohol as a solvent or an additive (promoter) in this reaction may remarkably improve the catalytic activity. The fluorine-containing alcohol is not especially limited and can be any aliphatic alcohol or aromatic alcohol in which part or all of hydrogen atoms in the hydrocarbon moiety are substituted with fluorine atoms. The fluorine-containing alcohol may be a monohydric alcohol or polyhydric alcohol.

Such fluorine-containing aliphatic alcohols include aliphatic chain alcohols and alicyclic alcohols. Preferred exemplary aliphatic chain alcohols include fluorine-containing linear aliphatic alcohols which are linear alcohols having about 1 to about 20 carbon atoms in which part or all of hydrogens in the hydrocarbon moiety are substituted with fluorine atoms; and fluorine-containing branched-chain aliphatic alcohols which are branched-chain alcohols having about 3 to about 20 carbon atoms in which part or all of hydrogens in the hydrocarbon moiety are substituted with fluorine atoms. In such fluorine-containing aliphatic chain alcohols, the hydrocarbon moiety (or fluorinated hydrocarbon moiety) may contain one or more unsaturated bonds. Specific examples of fluorine-containing linear aliphatic alcohols in which part of hydrogens in the hydrocarbon moiety are substituted with fluorine atoms include 1,1-difluoroethanol, 1,1,2-trifluoroethanol, 2,2,2-trifluoroethanol, 1,1-difluoro-1-propanol, 1,2-difluoro-1-propanol, 1,2,3-trifluoro-1-propanol, 3,3,3-trifluoro-1-propanol, 1,1,2,2-tetrafluoro-1-propanol, 1,3-difluoro-1,3-propanediol, 2,3,4-trifluoro-1-butanol, 4,4,4-trifluoro-1-butanol, 3,3,4,4,4-pentafluoro-1-butanol, 1,1,2,2,3,3-hexafluoro-1-butanol, 1,1,2,2-tetrafluoro-1-butanol, 1,2,3,4-tetrafluoro-1-butanol, 3,3,4,4,4-pentafluoro-1-butanol, 1,2,3,4-tetrafluoro-1,4-butanediol, 1,1,2,2-tetrafluoro-1-pentanol, 5,5,5-trifluoro-1-pentanol, 4,4,5,5,5-pentafluoro-1-pentanol, 1,1,2,2-tetrafluoro-1-hexanol, and 5,5,6,6,6-pentafluoro-1-hexanol. Exemplary fluorine-containing aliphatic branched-chain alcohols include hexafluoroisopropyl alcohol, heptafluoroisopropyl alcohol, 3,3,3-trifluoro-2-trifluoromethyl-1-propanol, 2-trifluoromethyl-1-butanol, 2-trifluoromethyl-1,4-butanediol, and 2-trifluoromethyl-3,3,4,4,4-pentafluoro-1-butanol.

Exemplary usable fluorine-containing alicyclic alcohols are those corresponding to alicyclic alcohols having about 3 to about 20 carbon atoms, such as cyclohexanol and cyclopentanol, except for containing one or more fluorine atoms per molecule. The way to contain such fluorine atom is not especially limited. For example, any of alicyclic alcohols containing a fluorine atom bonded to a carbon atom constituting the ring; and alicyclic alcohols containing a fluorine-containing hydrocarbon group bonded to a carbon atom constituting the ring will do.

Exemplary usable fluorine-containing aromatic alcohols are those corresponding to aromatic alcohols, such as benzyl alcohol and phenylethanol, except for containing one or more fluorine atoms per molecule. The way to contain fluorine atom is not especially limited, and any of aromatic alcohols having a fluorinated hydrocarbon group substituted on the aromatic ring; and aromatic alcohols having a chain hydrocarbon moiety containing fluorine atom will do.

The use of an acid in this reaction may remarkably increase the catalytic activity. The acid may be any of Lewis acids and Broensted acids. Exemplary Lewis acids include aluminum chloride, zinc chloride, and metal triflates. Exemplary Broensted acids include inorganic acids such as sulfuric acid, hydrochloric acid, and nitric acid; and organic acids including sulfonic acids such as p-toluenesulfonic acid and methanesulfonic acid.

The amount of acids to be added is, for example, from about 0.0001 to about 1 mole, preferably from about 0.0005 to about 0.5 mole, and more preferably from about 0.001 to about 0.2 mole, per 1 mole of the oxime compound. Each of different acids may be used alone or in combination.

When treated according to the process of the present invention, for example, an oxime compound represented by Formula (4) gives an amide compound represented by following Formula (6); and a cyclic oxime compound represented by Formula (5) gives a lactam represented by following Formula (7). More specifically, acetophenone oxime gives, for example, acetanilide; and a cycloalkanone oxime gives a corresponding lactam having one more member than the cycloalkanone. Typically, cyclohexanone oxime gives ε-caprolactam; cycloheptanone oxime gives 7-heptane lactam; cyclooctanone oxime gives 8-octane lactam; and cyclododecanone oxime gives 12-laurolactam. The groups R^{a} and R^{b} in Formula (6) and the repetition number "m" in Formula (7) are as defined above.

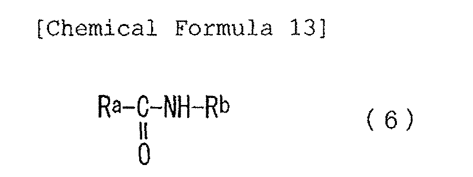

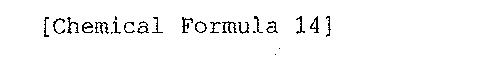

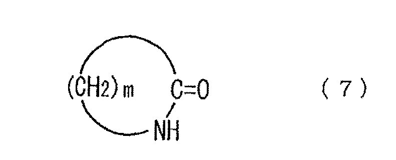

After the completion of the reaction, a reaction product can be separated and purified through a separation procedure such as filtration, concentration, distillation, extraction, crystallization, recrystallization, adsorption, or column chromatography, or any combination of them.

The oxime compound is very advantageously prepared by a process mentioned below, because it can be efficiently prepared in a simple manner under mild conditions; and, additionally, the reaction for synthesizing an oxime compound, and the reaction for producing an amide or lactam through rearrangement of the oxime compound can be carried out in one step without requiring an extra intermediate step for separating and purifying the oxime compound.

Specifically, the oxime compound is preferably prepared by allowing a compound having methyl group or methylene group to react with a nitrous ester or nitrite in the presence of a nitrogen-containing cyclic compound containing, as a ring constituent, a skeleton represented by following Formula (8):

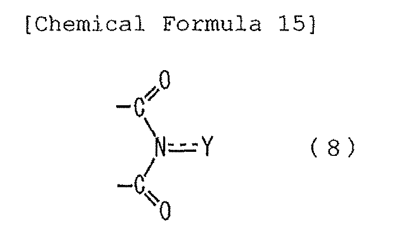

wherein Y represents an oxygen atom or -OR' group, where R' represents a hydrogen atom or hydroxyl-protecting group.

Specific usable examples of the nitrogen-containing cyclic compound containing a skeleton represented by Formula (8) as a ring constituent include N-hydroxy imide compounds derived from aliphatic polycarboxylic acid anhydrides (cyclic anhydrides) or aromatic polycarboxylic acid anhydrides (cyclic anhydrides), such as N-hydroxysuccinimide, N-hydroxyphthalimide, N,N'-dihydroxypyromellitic diimide, N-hydroxyglutarimide, N-hydroxy-1,8-naphthalenedicarboximide, and N,N'-dihydroxy-1,8,4,5-naphthalenetetracarboxylic diimide; and compounds corresponding to the N-hydroxy imide compounds, except with hydroxyl group being protected by a protecting group (e.g., an acyl group such as acetyl group).

Examples of the compound having methyl group or methylene group include compounds represented by following Formula (9):

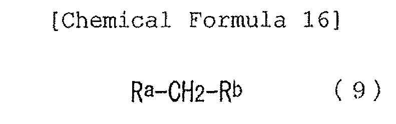

wherein R^{a} and R^{b} are as defined above.

Specific examples of the compounds of Formula (9) include ethane, propane, butane, pentane, hexane, heptane, octane, n-propylcyclohexane, toluene, p-xylene, ethylbenzene, isopropylbenzene, diphenylmethane, and 1,2-diphenylethane.

Exemplary compounds having methylene group further include compounds represented by following Formula (10):

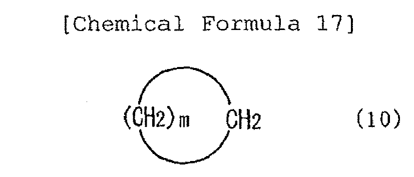

wherein "m" is as defined above.

The ring in Formula (10) may be bonded with a substituent and may be fused with another ring. Exemplary compounds represented by Formula (10) include cyclopropane, cyclobutane, cyclopentane, cyclohexane, cycloheptane, cyclooctane, cyclononane, cyclodecane, cyclododecane, cyclotridecane, cyclotetradecane, cyclopentadecane, cyclohexadecane, cyclooctadecane, and cyclononadecane. Exemplary substituents to which the ring may be bonded are as with the substituents which the organic group may have.

Exemplary nitrous esters include alkyl nitrites such as methyl nitrite, ethyl nitrite, propyl nitrite, isopropyl nitrite, butyl nitrite, isobutyl nitrite, t-butyl nitrite, amyl nitrite, isoamyl nitrite, t-amyl nitrite, and hexyl nitrite; aryl nitrites such as phenyl nitrite; and aralkyl nitrites such as benzyl nitrite. Preferred examples of nitrous esters include alkyl nitrites such as alkyl nitrites whose alkyl moiety having 1 to 6 carbon atoms. Exemplary nitrites (salts of nitrous acid) include ammonium nitrite; nitrites of alkaline earth metals, such as lithium nitrite, sodium nitrite, potassium nitrite, and barium nitrite; and nitrites of other metals, such as zinc nitrite.

The proportion between the compound having methyl group or methylene group and the nitrous ester or nitrite can be suitably set according typically to the types and combination of the two compounds. Typically, the compound having methyl group or methylene group may be used in an amount substantially equivalent or in excess (e.g., from about 1.1 to about 50 times by equivalent or more, and preferably from about 3 to about 30 times by equivalent); or contrarily, the nitrous ester or nitrite may be used in excess to the compound having methyl group or methylene group.

The reaction between the compound having methyl group or methylene group and the nitrous ester or nitrite is carried out in the presence of, or in the absence of, a solvent. The solvent is not especially limited and, for example, the solvents listed in the rearrangement reaction of the oxime compound can be used herein. The reaction temperature and other conditions are not especially limited, and the reaction herein can be carried out typically under the same or similar conditions to those in the rearrangement reaction of the oxime compound. Typically, the reaction temperature is from about 0°C to about 250°C, preferably from about 25°C to about 150°C, and more preferably from about 40°C to about 120°C. The reaction may be carried out in an atmosphere of an inert gas such as nitrogen or argon gas, but it can be carried out in an air atmosphere or oxygen atmosphere typically in the case of target products of some types. The reaction can be carried out under reduced pressure, under normal atmospheric pressure, or under a pressure (under a load), according to a common system or procedure such as a batch system, semi-batch system, or continuous system (e.g., multistage continuous circulation system). The yield is significantly improved when the reaction is carried out under reduced pressure, especially under such a reduced pressure that nitrogen oxide gases (particularly NO₂) produced as by-products as a result of the reaction can be removed from the system [e.g., from about 30 to about 700 mmHg (from about 3.99 to about 93.1 kPa)]. This is probably because nitrogen oxide gases such as NO₂ will inhibit the reaction.

It is considered that the reaction between the compound having methyl group or methylene group and the nitrous ester or nitrite initially gives a nitroso compound, and this compound is rearranged to give an oxime compound. For example, it is considered that the reaction between cyclohexane and a nitrous ester or nitrite initially gives nitrosocyclohexane, and this compound is rearranged to give cyclohexanone oxime. Though varying from type to type, a nitroso compound of some type may be in reversible equilibrium with a corresponding dimer (di-N-oxide compound in which two molecules of the nitroso compound are bonded through their nitrogen atoms) and the equilibrium may lie to the dimer. When the reaction is carried out over a long period of time, the nitroso compound and a dimer thereof can be in a trace amount, at most in a yield of less than 1%.

In a preferred embodiment, the reaction between the compound having methyl group or methylene group and the nitrous ester or nitrite is carried out by consecutively or continuously adding the nitrous ester or nitrite to the reaction system. According to this embodiment, side reactions particularly in nitrosation stage can be suppressed, and thereby the nitroso compound (or a dimer thereof) can be produced with a high selectivity, as compared to a process of adding the nitrous ester or nitrite at once. Thus, an oxime compound, for example, can be obtained in a high yield typically through the subsequent rearrangement reaction.

In another embodiment, for producing an oxime compound in a good yield, reactions are allowed to proceed stepwisely by independently providing the step of reacting a compound having methyl group or methylene group with a nitrous ester or nitrite to give a nitroso compound or a dimer thereof and the step of converting the resulting nitroso compound or a dimer thereof into an oxime compound. In this embodiment, the total reaction time can be significantly shortened by adding an additive to the reaction system or carrying out heating in the subsequent conversion step (rearrangement step of the nitroso compound). The subsequent rearrangement step may use another solvent than the solvent used in the precedent nitrosation step. In this embodiment, the precedent nitrosation step is preferably carried out under reduced pressure, because the yield is significantly improved for the same reason as above.

Though not especially limited, as long as being capable of inducing the rearrangement from a nitroso form to an oxime form, the additive is preferably selected typically from acids and bases. Exemplary acids herein include sulfonic acids such as methanesulfonic acid, trifluoromethanesulfonic acid, benzenesulfonic acid, and p-toluenesulfonic acid; mineral acids such as sulfuric acid, nitric acid, hydrochloric acid, phosphoric acid, boric acid, and fuming sulfuric acid; Lewis acids such as aluminum chloride, zinc chloride, and scandium triflate; solid acids such as silica, alumina, and zeolite; complex acids including polyacids such as phosphomolybdic acid, phosphotungstic acid, silicomolybdic acid, and silicotungstic acid; and strongly acidic cation-exchange resins, Exemplary bases include organic bases including tertiary amines such as triethylamine, nitrogen-containing heterocyclic compounds such as pyridine, as well as sodium acetate and sodium methoxide; inorganic bases such as sodium carbonate, sodium hydrogen carbonate, sodium hydroxide, and potassium hydroxide; and solid bases such as magnesium oxide, hydrotalcite, and hydroxyapatite. The additive(s) may be added at once or in two or more installments. The amount of additives is, for example, from about 0.01 to about 100 parts by weight, preferably from about 0.1 to about 50 parts by weight, and more preferably from about 0.3 to about 30 parts by weight, per 100 parts by weight of the compound having methyl group or methylene group. A rearrangement reaction using additives may be carried out at a temperature of, for example, from about 40°C to about 120°C, and preferably from about 50°C to about 100°C for a duration of, for example, from about 5 to about 180 minutes, and preferably from about 10 to about 120 minutes. A rearrangement reaction with heating may be carried out at a heating temperature of, for example, from about 120°C to about 250°C, and preferably from about 150°C to about 200°C for a reaction time of, for example, from about 0.5 to about 120 minutes, and preferably from about 2 to about 90 minutes.

In the production of an oxime compound, it is possible to produce a corresponding amide or lactam from a compound having methyl group or methylene group in one step, by simultaneously adding the compound containing a structure represented by Formula (1) (preferably, in combination with a fluorine-containing alcohol) in addition to the compound having methyl group or methylene group, nitrous ester or nitrite, and nitrogen-containing cyclic compound containing a skeleton represented by Formula (8) as a ring constituent and carrying out a reaction. In another possible process, a reaction between a compound having methyl group or methylene group and a nitrous ester or nitrite is carried out in the presence of a nitrogen-containing cyclic compound containing a skeleton represented by Formula (8) as a ring constituent and a compound containing a structure represented by Formula (1) to give an oxime compound, and thereafter a fluorine-containing alcohol is added, followed by carrying out a rearrangement reaction of the oxime compound. In yet another possible process, a reaction between a compound having methyl group or methylene group and a nitrous ester or nitrite is carried out in the presence of a nitrogen-containing cyclic compound containing a skeleton represented by Formula (8) as a ring constituent (preferably in combination with a fluorine-containing alcohol) to give an oxime compound, and thereafter a compound containing a structure represented by Formula (1) is added, followed by carrying out a rearrangement reaction of the oxime compound. In still another possible process, a reaction between a compound having methyl group or methylene group and a nitrous ester or nitrite is carried out in the presence of a nitrogen-containing cyclic compound containing a skeleton represented by Formula (8) as a ring constituent to give an oxime compound, and thereafter a compound containing a structure represented by Formula (1) and a fluorine-containing alcohol are added, followed by carrying out a rearrangement reaction of the oxime compound. In these processes, an operation such as distilling off of the solvent, concentration, or exchange of the solvent may be carried out in an appropriate stage. In a preferred embodiment, the used nitrogen-containing cyclic compound containing a skeleton represented by Formula (8) as a ring constituent is removed typically through precipitation and filtration before the Beckmann rearrangement reaction. The production of the oxime compound may be conducted stepwisely (step by step), as described above.

According to the processes of the present invention, amides or lactams can be produced in a simple manner and in a high yield without causing large amounts of by-products. Further, high-purity amides or lactams cab be produced in a simple manner, because the catalyst and other components for use in the present invention are easily separable from the product amides or lactams. Additionally, amides or lactams can be produced efficiently in a simple manner, because it is possible to carry out the step of producing an oxime from a raw material such as an aliphatic or aromatic hydrocarbon and the step of producing an amide or lactam form the oxime compound as one step or in one pot. Typically, ε-caprolactam and ω-laurolactam can be efficiently produced from cyclohexane and cyclododecane, respectively.

The product amides or lactams are very industrially important, because they can be used typically as raw materials for pharmaceutical drugs, agricultural chemicals, dyestuffs, solvents, and explosives; and as raw materials for polyamides (nylons).

### EXAMPLES

The present invention will be illustrated in further detail with reference to several examples below, which, however, are by no means intended to limit the scope of the present invention.

### EXAMPLE 1

In a reactor were placed cyclododecanone oxime (10 mmol), hexachlorophosphazene [a compound of Formula (1a) in which X, R¹, R², R³, R⁴, and R⁵ are each Cl; 1 percent by mole], and acetonitrile (20 mL), followed by stirring at 70°C for 2 hours. A gas chromatographic analysis was conducted after the reaction to find that laurolactam was produced in a yield of 78%.

### EXAMPLE 2

In a reactor were placed cyclododecanone oxime (10 mmol), hexachlorophosphazene (1 percent by mole), and hexafluoroisopropyl alcohol (20 mL), followed by stirring at 70°C for 2 hours. A gas chromatographic analysis was conducted after the reaction to find that laurolactam was produced in a yield of 99%.

### EXAMPLE 3

In a reactor were placed cyclododecanone oxime (10 mmol), hexachlorophosphazene (1 percent by mole), p-toluenesulfonic acid (5 percent by mole), and acetonitrile (20 mL), followed by stirring at 70°C for 2 hours. A gas chromatographic analysis was conducted after the reaction to find that laurolactam was produced in a yield of 91%.

### EXAMPLE 4

In a reactor were placed cyclododecanone oxime (10 mmol), hexachlorophosphazene (1 percent by mole), and pentafluoro-1-propanol (20 mL), followed by stirring at 70°C for 2 hours. A gas chromatographic analysis was conducted after the reaction to find that laurolactam was produced in a yield of 74%.

### EXAMPLE 5

In a reactor were placed cyclododecanone oxime (10 mmol), hexachlorophosphazene (1 percent by mole), and ethyl acetate (20 mL), followed by stirring at 70°C for 2 hours. A gas chromatographic analysis was conducted after the reaction to find that laurolactam was produced in a yield of 12%.

### EXAMPLE 6

In a reactor were placed cyclododecanone oxime (10 mmol), hexachlorophosphazene (1 percent by mole), and t-butyl alcohol (20 mL), followed by stirring at 70°C for 2 hours. A gas chromatographic analysis was conducted after the reaction to find that laurolactam was produced in a yield of 6%.

### EXAMPLE 7

In a reactor were placed cyclododecanone oxime (10 mmol), hexachlorophosphazene (1 percent by mole), and toluene (20 mL), followed by stirring at 70°C for 2 hours. A gas chromatographic analysis was conducted after the reaction to find that laurolactam was produced in a yield of 4%.

### EXAMPLE 8

In a reactor were placed cyclododecanone oxime (10 mmol), N-bromosuccinimide (10 percent by mole), and hexafluoroisopropyl alcohol (20 mL), followed by stirring at 70°C for 2 hours. A gas chromatographic analysis was conducted after the reaction to find that laurolactam was produced in a yield of 95%.

### EXAMPLE 9

In a reactor were placed cyclododecanone oxime (10 mmol), N-chlorosuccinimide (10 percent by mole), and hexafluoroisopropyl alcohol (20 mL), followed by stirring at 70°C for 2 hours. A gas chromatographic analysis was conducted after the reaction to find that laurolactam was produced in a yield of 98%.

### EXAMPLE 10

In a reactor were placed cyclododecanone oxime (10 mmol), N-chlorophthalimide (10 percent by mole), and hexafluoroisopropyl alcohol (20 mL), followed by stirring at 70°C for 2 hours. A gas chromatographic analysis was conducted after the reaction to find that laurolactam was produced in a yield of 95%.

### EXAMPLE 11

In a reactor were placed cyclododecanone oxime (10 mmol), isocyanuric chloride (1 percent by mole), and hexafluoroisopropyl alcohol (20 mL), followed by stirring at 70°C for 2 hours. A gas chromatographic analysis was conducted after the reaction to find that laurolactam was produced in a yield of 96%.

### EXAMPLE 12

In a reactor were placed cyclododecanone oxime (10 mmol), diethoxyphosphoryl chloride (5 percent by mole), and hexafluoroisopropyl alcohol (20 mL), followed by stirring at 70°C for 2 hours. A gas chromatographic analysis was conducted after the reaction to find that laurolactam was produced in a yield of 94%.

### EXAMPLE 13

In a reactor were placed cyclododecanone oxime (10 mmol), isocyanuric chloride (1 percent by mole), and acetonitrile (20 mL), followed by stirring at 70°C for 2 hours. A gas chromatographic analysis was conducted after the reaction to find that laurolactam was produced in a yield of 76%.

### EXAMPLE 14

In a reactor were placed cyclododecanone oxime (10 mmol), isocyanuric chloride (1 percent by mole), and cyclododecane (20 g), followed by stirring at 70°C for 2 hours. A gas chromatographic analysis was conducted after the reaction to find that laurolactam was produced in a yield of 56%.

### EXAMPLE 15

In a reactor were placed cyclohexanone oxime (10 mmol), isocyanuric chloride (10 percent by mole), and hexafluoroisopropyl alcohol (20 mL), followed by stirring at 70°C for 2 hours. A gas chromatographic analysis was conducted after the reaction to find that caprolactam was produced in a yield of 15%.

### EXAMPLE 16

In a reactor were placed cyclohexanone oxime (10 mmol), hexafluorophosphazene (10 percent by mole), and hexafluoroisopropyl alcohol (20 mL), followed by stirring at 70°C for 2 hours. A gas chromatographic analysis was conducted after the reaction to find that caprolactam was produced in a yield of 26%.

### EXAMPLE 17

In a reactor were placed cyclododecanone oxime (10 mmol), triphenylchlorosilane (10 percent by mole), and hexafluoroisopropyl alcohol (20 mL), followed by stirring at 70°C for 2 hours. A gas chromatographic analysis was conducted after the reaction to find that laurolactam was produced in a yield of 9%.

### EXAMPLE 18

In a reactor were placed cyclododecanone oxime (10 mmol), triphenylchlorosilane (10 percent by mole), and hexafluoroisopropyl alcohol (20 mL), followed by stirring at 70°C for 2 hours. A gas chromatographic analysis was conducted after the reaction to find that laurolactam was produced in a yield of 9%.

### EXAMPLE 19

In a reactor were placed cyclododecanone oxime (10 mmol), toluenesulfonyl chloride (10 percent by mole), and hexafluoroisopropyl alcohol (20 mL), followed by stirring at 70°C for 2 hours. A gas chromatographic analysis was conducted after the reaction to find that laurolactam was produced in a yield of 23%.

### EXAMPLE 20

Cyclododecane (100 g, 0.595 mol), N-hydroxyphthalimide (1.94 g, 0.0119 mol), and acetic acid (80 g) were placed in a flask, oxygen was purged therefrom, the mixture was raised in temperature to 70°C in a nitrogen gas atmosphere (1 atm = 0.101 MPa), and t-butyl nitrite (12.5 g, 0.119 mol) was added over 2 hours. Thirty (30) minutes later, acetic acid was removed at the same temperature (70°C) under reduced pressure, heptane (100 g) was added to precipitate N-hydroxyphthalimide, and the precipitated N-hydroxyphthalimide was separated by filtration. Heptane was then removed by distillation, and triethylamine (3.61 g, 3.57 mmol) was added, followed by stirring at 70°C for 2 hours. Next, triethylamine was removed, and hexachlorophosphazene (2.98 mmol) and hexafluoroisopropyl alcohol (6 mmol) were added, followed by stirring at 80°C for 2 hours. A gas chromatographic analysis was conducted after the reaction to find that laurolactam was produced in a yield on the basis of cyclododecane of 12% with a conversion of 17%.

### EXAMPLE 21

Cyclododecane (100 g, 0.595 mol), N-hydroxyphthalimide (1.94 g, 0.0119 mol), and acetic acid (80 g) were placed in a flask, oxygen was purged therefrom, the mixture was raised in temperature to 70°C in a nitrogen gas atmosphere (1 atm = 0.101 MPa), and t-butyl nitrite (12.5 g, 0.119 mol) was added over 2 hours. Thirty (30) minutes later, acetic acid was removed at the same temperature (70°C) under reduced pressure, heptane (100 g) was added to precipitate N-hydroxyphthalimide, and the precipitated N-hydroxyphthalimide was separated by filtration. Heptane was then removed by distillation, and triethylamine (3.61 g, 3.57 mmol) was added, followed by stirring at 70°C for 2 hours. Next, triethylamine was removed, isocyanuric chloride (2.98 mmol) and hexafluoroisopropyl alcohol (6 mmol) were added, followed by stirring at 80°C for 2 hours. A gas chromatographic analysis was conducted after the reaction to find that laurolactam was produced in a yield on the basis of cyclododecane of 11% with a conversion of 17%.

### EXAMPLE 22

Cyclododecane (100 g, 0.595 mol), N-hydroxyphthalimide (1.94 g, 0.0119 mol), and acetic acid (80 g) were placed in a flask, oxygen was purged therefrom, the mixture was raised in temperature to 70°C in a nitrogen gas atmosphere (1 atm = 0.101 MPa), and t-butyl nitrite (12.5 g, 0.119 mol) was added over 2 hours. Thirty (30) minutes later, acetic acid was removed at the same temperature (70°C) under reduced pressure, heptane (100 g) was added to precipitate N-hydroxyphthalimide, and the precipitated N-hydroxyphthalimide was separated by filtration. Heptane was then removed by distillation, and triethylamine (3.61 g, 3.57 mmol) was added, followed by stirring at 70°C for 2 hours. Next, triethylamine was removed, and N-chlorosuccinimide (5 mmol) and hexafluoroisopropyl alcohol (20 mmol) were added, followed by stirring at 80°C for 2 hours. A gas chromatographic analysis was conducted after the reaction to find that laurolactam was produced in a yield on the basis of cyclododecane of 12% with a conversion of 17%.

### EXAMPLE 23

Cyclododecane (100 g, 0.595 mol), N-hydroxyphthalimide (1.94 g, 0.0119 mol), and acetic acid (80 g) were placed in a flask, oxygen was purged therefrom, the mixture was raised in temperature to 70°C in a nitrogen gas atmosphere (1 atm = 0.101 MPa), and t-butyl nitrite (12.5 g, 0.119 mol) was added over 2 hours. Thirty (30) minutes later, acetic acid was removed at the same temperature (70°C) under reduced pressure, heptane (100 g) was added to precipitate N-hydroxyphthalimide, and the precipitated N-hydroxyphthalimide was separated by filtration. Heptane was then removed by distillation, and triethylamine (3.61 g, 3.57 mmol) was added, followed by stirring at 70°C for 2 hours. Next, triethylamine was removed, and isocyanuric chloride (2.98 mmol) was added, followed by stirring at 80°C for 2 hours. A gas chromatographic analysis was conducted after the reaction to find that laurolactam was produced in a yield on the basis of cyclododecane of 3% with a conversion of 17%.

### EXAMPLE 24

In a reactor were placed cyclododecanone oxime (10 mmol), hexachlorophosphazene (1 percent by mole), and acetonitrile (20 mL), followed by stirring at 90°C for 2 hours. A gas chromatographic analysis was conducted after the reaction to find that laurolactam was produced in a yield of 99%.

### EXAMPLE 25

In a reactor were placed cyclododecanone oxime (10 mmol), hexachlorophosphazene (0.5 percent by mole), and acetonitrile (20 mL), followed by stirring at 90°C for 2 hours. A gas chromatographic analysis was conducted after the reaction to find that laurolactam was produced in a yield of 96%.

### EXAMPLE 26

In a reactor were placed cyclododecanone oxime (10 mmol), diethoxyphosphoryl chloride (5 percent by mole), p-toluenesulfonic acid (5 percent by mole), and acetonitrile (20 mL), followed by stirring at 70°C for 2 hours. A gas chromatographic analysis was conducted after the reaction to find that laurolactam was produced in a yield of 56%.

### EXAMPLE 27

In a reactor were placed cyclopentanone oxime (10 mmol), hexachlorophosphazene (1 percent by mole), and hexafluoroisopropyl alcohol (20 mL), followed by stirring at 70°C for 2 hours. A gas chromatographic analysis was conducted after the reaction to find that valerolactam was produced in a yield of 11%.

### Industrial Applicability

According to the present invention, amides or lactams can be produced in a high yield and in a simple manner through rearrangement reactions of oximes, and problems of removal and disposal of by-products occurring in known processes for producing amide or lactams can be avoided. The product amides or lactams are useful typically as raw materials for pharmaceutical drugs, agricultural chemicals, dyestuffs, solvents, and explosives; and as raw materials for polyamides (nylons).

## Claims

1. A process for producing an amide or lactam, the process comprising the step of carrying out rearrangement of an oxime compound in the presence of a compound containing a structure represented by following Formula (1):
-Z-X (1)
wherein Z represents phosphorus (P), nitrogen (N), sulfur (S), boron (B), or silicon (Si) atom; and X represents a leaving group, where Z is bonded to one or more atoms or groups besides X,
to give a corresponding amide or lactam.

2. The process for producing an amide or lactam, according to claim 1, wherein X is a halogen atom.
